# EUROPÄISCHE PATENTSCHRIFT

(11) **EP 0 981 508 B1**
(45) Veröffentlichungstag und Bekanntmachung des Hinweises auf die Patenterteilung: **06.02.2002**
(21) Anmeldenummer: 98922801.0
(22) Anmeldetag: 05.05.1998
(51) Int. Cl.: C07C 37/52

(54) **VERFAHREN ZUR AUFARBEITUNG VON MUTTERLAUGEN AUS DER BISPHENOLHERSTELLUNG**
METHOD FOR PROCESSING MOTHER LIQUORS FROM THE PRODUCTION OF BISPHENOL
PROCEDE DE TRAITEMENT DE LESSIVES-MERES EVACUEES LORS DE LA PRODUCTION DE BISPHENOLS

(30) Priorität: 16.05.1997 DE 19720540
(43) Veröffentlichungstag der Anmeldung: 01.03.2000
(73) Patentinhaber: Bayer Aktiengesellschaft, 51368 Leverkusen (DE)
(72) Erfinder: FENNHOFF, Gerhard, D-47877 Willich (DE); BUYSCH, Hans-Josef, D-47809 Krefeld (DE); FENGLER, Gerd, D-47802 Krefeld (DE)
(86) Internationale Anmeldenummer: EP9802642
(87) Internationale Veröffentlichungsnummer: WO9852897

(56) Entgegenhaltungen:
- BE-A- 678 415
- US-A- 4 131 749
- US-A- 4 351 966

## Beschreibung

Vorliegende Erfindung betrifft ein Verfahren zur Aufarbeitung von bei der Herstellung von Dihydroxydiarylalkanen (Bisphenolen) nach Abtrennung des Bisphenols verbleibenden, aus dem Verfahren ausgeschleusten Mutterlaugen.

Es ist bekannt, Bisphenole herzustellen durch sauer katalysierte Umsetzung von Ketonen mit Phenol. Dazu gibt es eine Reihe unterschiedlicher Vorschläge (z.B. US-A 2 775 620, EP-A 342 758, EP-A 616 993, EP-A 342 758, DE-OS 3 833 900, US-A 4 308 404, US-A 4 308 405, EP-A 630 878, US-A 4 876 391, US-A 3 242 219).

In Ullmann's Encyclopedia of Indust. Chem., 5. Ed., Vol. A 19, p. 348-52 findet sich eine Übersicht über die ältere Literatur zur Bisphenolherstellung. In der Regel werden Bisphenole, besonders Bisphenol A (BPA), das technisch wichtigste der Bisphenole, so hergestellt, daß man Keton und Phenol zu einer im Kreis geführten Mutterlauge aus der Bisphenol-Aufarbeitung hinzusetzt, dieses Gemisch über saure Ionenaustauscher leitet und die Umsetzung zu Bisphenol vollzieht. Aus dem Reaktionsgemisch wird gegebenenfalls nicht umgesetztes Keton zurückgewonnen und in die Reaktion zurückgeleitet. Das Reaktionsgemisch wird abgekühlt, man läßt das Bisphenol gegebenenfalls als Phenoladdukt auskristallisieren, trennt ab und wäscht mit Phenol. Aus dem Addukt wird durch Flashdestillation das Phenol abgetrennt und reines Bisphenol gewonnen. Die Mutterlauge der Kristallisation wird über saure lonenaustauscher geleitet und darin befindliche Isomere zu Bisphenol umgelagert. Das so entstandene Bisphenol wird per Kristallisation abgetrennt und das erhaltene Kristallisat in den ersten Kristallisationsschritt eingeschleust. Aus der nun erhaltenen Mutterlauge wird ein Teil (ca. 10 bis 20 %) abgezweigt, die Hauptmenge wird in die Reaktion zurückgeführt. Aus dem abgezweigten Teil wird Phenol abdestilliert und in die Reaktion geführt. Den bei der Destillation anfallenden Rückstand schleust man aus den Verfahren aus und verwendet ihn z.B. zur Herstellung von Phenolharz.

Man verliert eine nennenswerte Menge an wertvollen Verbindungen wie Bisphenol und Isomere mit dem abgezweigten und schließlich ausgeschleusten Teil Diese Entnahme aus der Mutterlauge ist aber unbedingt erforderlich, um die Menge an unbrauchbaren und störenden Verbindungen nicht zu groß werden zu lassen. Man hat daher versucht, den ausgeschleusten Anteil aufzuarbeiten und zu spalten, um die Wertstoffe für die Bisphenolherstellung zurückzugewinnen. Auch dazu gibt es eine Reihe von Vorschlägen.

Man kann durch Pyrolyse bei ca. 300°C Phenole und Alkenylphenole, die noch gründlich gereinigt werden müssen, in mäßiger Ausbeute erhalten (US-A 2 497 503). Auch eine hydrierende Behandlung führt zu Wertprodukten, wie EP-A 17 852 lehrt. Die Spaltung kann auch durch saure und basische Verbindungen beschleunigt werden. Mit Säuren wie Schwefel- oder Toluolsulfonsäure erhält man allerdings nur Phenol (US-A 3 466 337). Basische Katalysatoren dagegen bewirken eine Spaltung der ausgeschleusten Materialien in Phenol und Isoalkenylphenol. Als Katalysatoren werden genannt Alkaliverbindungen wie NaOH, KOH, NaHCO₃, Na-Acetat, Na-Hypophosphit, K₂CO₃, MgO und AI-Isopropylat (US-A 4 277 628, US-A 4 594 459, US-A 4 131 749).

Bei dieser Vorgehensweise wird allerdings nur ein Teil des ausgeschleusten Materials gespalten, und man arbeitet diskontinuierlich oder halbkontinuierlich. Vollkontinuierliche Verfahren sind unbekannt.

Diese Verfahren sind im Hinblick auf eine höhere Reinheit des Bisphenols dahingehend verbessert worden, daß man das Phenol/Isoalkenylphenol-Gemisch aus der Spaltung in die erste Mutterlauge nach Abtrennung der ersten Partie Bisphenol einspeist, das Gemisch über den sauren Katalysator leitet und die Umsetzung von Isoalkenylphenol mit Phenol und die Umlagerung gleichzeitig ablaufen läßt. Aus dem Gemisch wird dann die zweite Partie Bisphenol durch Kristallisation abgetrennt und die zweite Mutterlauge wieder zur Spaltung gegeben. Die zweite Bisphenol-Partie wird in die erste Kristallisation gegeben. Dadurch wird zwar die Reinheit des Bisphenols etwas erhöht, aber man kompliziert das Verfahren um einen weiteren Kreislauf (US-A 4 954 661).

Vorgeschlagen wurde auch, die zweite Mutterlauge im oben beschriebenen Bisphenolprozess oder die erste Mutterlauge nach Abtrennen der ersten Bisphenolmenge und nach der Umlagerung zumindestens teilweise destillativ aufzuarbeiten, um die darin enthaltenen Wertprodukte besser zu nutzen. Das dabei erhaltene Bisphenol wird in die erste Kristallisationsstufe zurückgegeben und dort gereinigt. Die Isomere enthaltenden Leichtsieder führt man der Reaktion zu (WO 94/20445 und EP-A 552 518). Die Schwierigkeit besteht in jedem Fall darin, aus der an Isomeren und Nebenprodukten stark angereicherten Mutterlauge mit vertretbarem Aufwand hinreichend reine Fraktionen zu erhalten, die besonders arm an den schwer abtrennbaren Chromanen und Indanen sind, ohne daß man den nicht mehr brauchbaren Rest zu sehr vergrößert und die Ausbeute schmälert. Deswegen muß ein Teil der Destillationsprodukte verworfen werden.

Die bei der Destillation erhaltenen Bisphenol-armen Fraktionen kann man auch der Spaltung unterwerfen und die Spaltprodukte in das Verfahren wieder einführen (EP-A 332 203).

Es wurde nun gefunden, daß man bei der Bisphenolsynthese nach dem eingangs beschriebenen Prozeß auf einfache Weise die Ausbeute erhöhen kann, indem man den auszuschleusenden Teil der zweiten Mutterlauge nach Abdestillieren von Phenol mit einem basischen Katalysator vermischt und als Schmelze kontinuierlich in eine Reaktivrektifikation eindosiert. In der Reaktivrektifikation wird diese Schmelze gespalten in ein über Kopf gehendes Gemisch aus Phenol und Isoalkenylphenol, das in die Reaktion fließt und einen Sumpf, der nach Sauerstellen in eine zweite Reaktivrektifikation dosiert wird, wo er gespalten wird in Phenol, das, gegebenenfalls nach weiterer Reinigung, in die Reaktion zurückgeführt wird und in einen zweiten Sumpf, der entsorgt wird.

Geeignete aromatische Hydroxyverbindungen für die Herstellung der Edukte für das erfindungsgemäße Verfahren sind in p-Position nicht substituiert und enthalten keine Substituenten zweiter Ordnung wie Cyano-, Carboxy- oder Nitrogruppen; genannt seien beispielsweise Phenol, o- und m-Kresol, 2,6-Dimethylphenol, o-tert.-Butylphenol, 2-Methyl-6-tert.-Butylphenol, o-Cyclohexylphenol. o-Phenylphenol, o-Isopropylphenol, 2-Methyl-6-cyclopentyl-phenol, o- und m-Chlorphenol, 2,3,6-Trimethylphenol. Bevorzugt sind Phenol, o- und m-Kresol, 2,6-Dimethylphenol, o-tert.-Butylphenol und o-Phenyl-phenol; besonders bevorzugt ist Phenol.

Geeignete Ketone enthalten wenigstens eine aliphatische Gruppe an der Carbonylfunktion; genannt seien beispielsweise Aceton, Methylethylketon, Methylpropylketon, Methylisopropylketon, Diethylketon, Acetophenon, Cyclo-hexanon, Cyclopentanon, Methyl-, Dimethyl- und Trimethylcyclohexanone, die auch geminale Methylgruppen aufweisen können wie 3,3-Dimethyl-5-methylcyclohexanon (Hydroisophoron). Bevorzugt sind Aceton, Acetophenon, Cyclohexanon und dessen Methylgruppen tragende Homologe; besonders bevorzugt ist Aceton.

Edukte für das erfindungsgemäße Verfahren sind die bei der Herstellung von Dihydroxydiarylalkanen durch Umsetzung von aromatischen Hydroxyverbindungen mit Ketonen und Isoalkenylphenolen an sauren Katalysatoren anfallenden Mutterlaugen.

Bevorzugte Edukte für das erfindungsgemäße Verfahren sind insbesondere auch die nach Abtrennung der Bis(4-hydroxyaryl)alkane verbleibenden Mutterlaugen, die nach Zugabe der verbrauchten Hydroxyverbindungen und gegebenenfalls Entnahme eines gewissen Anteils zur Vermeidung der Anreicherung unerwünschter Beiprodukte in das Verfahren zurückgeführt werden; für den Fall der Synthese von Bisphenol A enthalten solche Mutterlaugen ca. 78-88 Gew.-% Phenol und 22-12 Gew.-% Bisphenol A und Beiprodukte, die sich wie folgt zusammensetzen:

| | |
|---|---|
| Bisphenol A | 40-65 Gew.-% |
| o,p-Bisphenol | 14-19 Gew.-% |
| Trisphenol | 2-6 Gew.-% |
| Chromane | 4-17 Gew.-% |
| 1,3,3-Trimethyldihydroxyphenylindane | 3-13 Gew.-% |
| weitere Nebenprodukte | 3-15 Gew.-% |

Geeignete Katalysatoren für die basische Spaltung sind die in der Literatur genannten, bevorzugt Alkalioxide und -hydroxide, besonders bevorzugt NaOH und KOH. Sie werden im erfindungsgemäßen Verfahren in die Schmelze der Spaltungsedukte eingetragen, gelöst und homogen verteilt, wobei die Temperatur zweckmäßig zwischen 100 und 200°C, bevorzugt 120 und 180°C liegt.

Geeignete Katalysatoren für die saure Spaltung sind die in der Literatur angeführten, bevorzugt Schwefelsäure, Phosphorsäure, phosphorige Säure, deren partielle Salze wie NH₄HSO₄, NaHSO₄, KHSO₄, NaH₂PO₄, KH₂PO₄, NH₄H₂PO₄, NH₄H₂PO₃, KH₂PO₃ und Analoga, ferner die organischen Abkömmlinge dieser Säuren, nämlich aromatische Sulfonsäuren und Disulfonsäuren wie Benzol-, Toluol-, Xylol-, Phenolsulfonsäure, Diphenyldisulfonsäure, Diphenyletherdisulfonsäure, sodann aromatische Phosphon- und Phosphinsäuren wie Benzol-, Toluol-, Xylolphosphon- und -phosphinsäure, Diphenyl-diphosphon- und -diphosphinsäure, ferner feste Säuren wie saure Aluminiumoxide, Tonerden wie Bentonite und Montmorillonite, Zeolithe, Titan- und Zirkonoxide, Niob- und Tantaloxiden, bevorzugt sind saure Tonerden.

Die Mengen an Katalysatoren, die dem zu spaltenden Gemisch zugegeben werden, liegen zwischen 0,01 Gew.-% und 5 Gew.-%, bevorzugt 0,05 bis 3 Gew.-%, besonders bevorzugt 0,1 bis 2 Gew.-%, bezogen auf die Menge an Spaltgemisch.

Um diese Mengenregel für saure Katalysatoren einhalten zu können, müssen die basischen Katalysatoren nach der basischen Spaltung entweder aus dein Sumpf entfernt werden, was in aller Regel zu aufwendig sein dürfte, oder sie müssen zuerst durch eine starke Säure neutralisiert werden. Dann kann der Zusatz der erforderlichen Menge an saurem Katalysator erfolgen. Auf diese Weise ist sichergestellt, daß hinreichende Mengen aktiver Säure vorliegen. Sauerstellen im Sinne der Erfindung bedeutet also, daß man dem Sumpf der basischen Spaltung soviel an Säure zugibt, daß sowohl der darin befindliche basische Katalysator neutralisiert wird als auch darüber hinaus eine hinreichende Menge an saurem Katalysator für die folgende saure Spaltung zur Verfügung steht.

Die Katalysatoren können beispielsweise gelöst oder suspendiert in Phenol kontinuierlich dem in die Spaltkolonnen fließenden Strom zudosiert werden. Es ist jedoch auch möglich, sie in Zwischenbehältern diskontinuierlich dem zu spaltenden Material beizumischen und dann dieses Gemisch kontinuierlich in die Spaltkolonne zu fördern

Die Kolonnen, in denen die Reaktivrektifikationen durchgeführt werden, entsprechen im allgemeinen üblichen Destillationskolonnen. In den Kolonnen ist der Spaltung eine destillative und fraktionierende Trennung der Spaltprodukte Phenol und Isoalkenylphenol von den nicht gespaltenen oder auch nicht spaltbaren Verbindungen überlagert. Die Spaltprodukte gehen dabei, im allgemeinen schon rein genug für ihre weitere Verwendung, über Kopf Die nicht spaltbaren Anteile werden als Sumpf ausgetragen. Reaktivrektifikationen sind dem Fachmann bekannt und beispielsweise in Ullmann's Encyclopedia of Indust. Chem., 5. Ed., Vol. B 4, p. 321-8 beschrieben.

Im vorliegenden Fall kann es vorteilhaft sein, in den Spaltkolonnen entsprechend dem während der Spaltung von oben nach unten in der Kolonne abnehmenden Flüssigkeits- und Gasvolumen den Kolonnendurchmesser im unteren Teil angemessen abnehmen zu lassen.

Für die Einstellung einer geeigneten Verweilzeit des Spaltmaterials in der Kolonne ist es zweckmäßig, Böden in die Spaltkolonnen einzuziehen. Dies gilt besonders für den Teil, in dem die Spaltung abläuft. Im oberen Teil kann für eine effektive Trennung der Produkte die Verwendung von Füllkörpern oder Packungen angezeigt sein.

Für den Fall, daß die Reinheit des aus der zweiten Spaltkolonne abdestillierenden Phenols für die Veiwendung in der Bisphenolsynthese nicht ausreichend ist, kann man es in eine weitere Kolonne oder aber in die erste Spaltkolonne einführen und dort weiter reinigen.

Das erfindungsgemäße Verfahren läßt sich besonders vorteilhaft mit hochselektiven Prozessen zur Bisphenolherstellung kombinieren. Diese zeichnen sich dadurch aus, daß man bei möglichst niedriger Ketonkonzentration und niedriger Temperatur arbeitet. Dies erreicht man, indem man Phenol und in die Reaktion zurückzurührende Mutterlauge mit Keton und Isoalkenylphenol in mindestens zwei in Reihe geschalteten Reaktoren umsetzt, die saure Ionenaustauscher enthalten und die, beginnend bei möglichst tiefer, aber in Richtung zunehmenden Umsatzes steigender Temperatur betrieben werden, wobei die Gesamtmenge an Keton und Isoalkenylphenol auf die einzelnen Reaktoren aufgeteilt und vor dem Eintreten in den jeweiligen Reaktor homogen im Reaktionsgemisch verteilt wird.

Vor den einzelnen Reaktoren sind zweckmäßig sowohl Mischorgane bekannter Bauart angebracht, um die homogene Verteilung von Keton und Isalkenylphenol im Ausgangs- bzw. Reaktionsgemisch zu gewährleisten als auch Wärmetauscher zur gewünschten Temperierung des Gemisches vor dein Durchgang durch das Katalysatorbett.

Nach dem Verlassen der Reaktoren wird zunächst nicht umgesetztes Keton abdestilliert und dann die erste Kristallisation vorgenommen. Die Kristalle werden abgetrennt und mit Phenol gewaschen; anschließend wird das Bisphenol isoliert. Die Mutterlauge wird von Wasser befreit und durch den Umlagerungsreaktor geschickt. Aus der Reaktionsmischung nach der Umlagerung wird eine zweite Partie Bisphenol kristallisiert, abgetrennt, gewaschen und in die erste Kristallisation gegeben. Die Mutterlauge der zweiten Kristallisation wird geteilt und zu 80 bis 90 % in die Reaktion zurückgeleitet.

Die restlichen 10 bis 20 % werden eingedampft, das destillierte Phenol wird in die Reaktion oder Wäsche geleitet. Der verbleibende Rückstand, enthaltend Bisphenol, Isomere und Nebenprodukte verschiedener Art wird als Schmelze nach Vermischen mit basischen Katalysatoren vollkontinuierlich in eine Reaktivrektifikation geleitet und darin bei Sumpftemperaturen von 190 bis 270°C, bevorzugt 200 bis 260°C, besonders bevorzugt 210 bis 250°C und Drücken von 15 bis 0,5 mbar, bevorzugt 12 bis 1 mbar, besonders bevorzugt 10 bis 1 mbar gespalten. Das Destillat, bestehend aus Phenol und Isoalkenylphenol und dessen Oligomeren wird der Reaktion zugeleitet, der Sumpf nach Sauerstellen in eine zweite, der vorgenannten ähnlichen Reaktivrektifikation eindosiert und darin bei 150 bis 260°C, bevorzugt 160 bis 250°C, besonders bevorzugt 170 bis 240°C und den oben angegebenen Drücken gespalten. Dabei destilliert Phenol ab, das gegebenenfalls über eine Kolonne weiter gereinigt werden kann und dem Reaktionsstrom beigemischt wird. Am Fuß der Kolonne wird ein Sumpf entnommen, der zu entsorgen ist.

Wenn der Sumpfanteil aus der basischen Spaltung aufgrund eines relativ kleinen Produktionsstranges einer Bisphenol-Anlage für den ökonomischen Betrieb einer Kolonne zu klein ist, empfiehlt sich eine Zusammenführung solcher Kleinmengen aus mehreren Strängen oder eine Sammlung des Sumpfes aus einer Anlage zu einer größeren Menge und dann deren Spaltung in einer geeigneten Apparatur.

Nach dem erfindungsgemäßen Verfahren lassen sich in vollkontinuierlicher, einfacher und apparativ wenig aufwendiger Arbeitsweise die Materialausbeuten bei der Bisphenolproduktion beträchtlich erhöhen.

### Beispiel

Ein Harz aus einer üblichen Bisphenol A-Produktion, entstanden nach Abtrennung von BPA, Umlagerung, einer zweiten BPA-Abtrennung und Abdestillieren von Phenol mit folgender Zusammensetzung:

| | |
|---|---|
| 0,5 % | Phenol |
| 0,90 % | Dimethylxanthen |
| 0,33 % | o,o'-Bisphenol |
| 17,74 % | o,p'-Bisphenol |
| 6,31 % | Chromane |
| 56,31 % | BPA |
| 7,05 % | Indane |
| 0,90 % | Spirobisindane |
| 4,37 % | Trisphenol |
| 1,18 % | Trihydroxychromanc (MG 402) |
| 4,26 % | unbekannte Verbindungen |

wurde nach Aufschmelzen und Zugabe von 0,3 Gew.-% KOH mit einer Temperatur von 145°C kontinuierlich auf eine 10bödige Kolonne mit einem Flüssigkeitsinhalt (Holdup) von 370 ml mit einer Menge von 400 g/h gegeben. Die Kolonne war mit einer auf 180°C temperierten Heizung umgeben, am Fuß mit einem auf 235°C temperierten Sumpf und am Kopf mit einer 50 cm langen verspiegelten Vigreux-Kolonne als Trennapparat versehen. Während des Betriebes bei 2 mbar ging kontinuierlich aus der Reaktivkolonne über die Vigreux-Kolonne ein Destillat über und ein Teil der eindosierten Harzmenge floß in den Sumpfkolben, aus dem kontinuierlich soviel Material abgezogen wurde, daß er etwa zur Hälfte gefüllt blieb. Nach mehreren Stunden erhielt man ein Destillat der Zusammensetzung

| | |
|---|---|
| 50,4 % | Phenol |
| 0,03 % | o,o'-Bisphenol |
| 0,04 % | o,p'-Bisphenol |
| 0,3 % | Chromane |
| 1,2 % | Dimethylxanthen |
| 1,3 % | unbekannte Komponenten |
| Rest | p-Isopropenylphenol und dessen Oligomere, |

das etwa 50 % der zudosierten Harzmenge entsprach und entsprechend etwa 50 Gcw. -% Rückstand aus dem Sumpfkolben.

Von diesem Rückstand wurde eine größere Menge gesammelt, mit soviel Schwefelsäure versetzt, daß die vorhandene KOH in KHSO₄ übergeführt wurde, außerdem wurden 0,9 Gew.-% saures Schichtsilikat (Tonsil Optimum), bezogen auf die Rückstandsmenge, zugesetzt, verrührt und analog dem BPA-Harz in der oben beschriebenen Apparatur gespalten, wobei die Kolonne auf ~150°C und die Sumpftemperatur auf 180 bis 190°C eingestellt wurden. Dabei gingen kontinuierlich 62 % der zudosierten Rückstandsmenge als Destillat über mit der Zusammensetzung

| | |
|---|---|
| 91,5% | Phenol |
| 0,02 % | o,o'-Bispshenol |
| 0,14 % | o,p-Bisphenol |
| 1,90 % | Chronnane |
| 0,01 % | Dimethylxanthen |
| 0,04 % | BPA |
| 0,17 % | Indan |
| 0,41 % | Spirobisindan |
| 5,8 % | unbekannte Verbindungen |

Der Rückstand dieser zweiten Spaltung betrug demnach 38 Gew.-% des ersten Rückstandes und 19 Gew.-% des ursprünglich eingesetzten BPA-Harzes. Unter der Voraussetzung, daß bei einer BPA-Produktion von 100 000 jato je nach Arbeitsweise 5 000 bis 6 000 jato Harz anfallen, kann nach dem erfindungsgemäßen Verfahren die Menge an Abfall auf 1000 bis 1200 jato reduziert und bei Verwendung der Spaltprodukte zur BPA-Synthese die Ausbeute an BPA um 4000 bis 4800 jato entsprechend ca. 4 bis 5 % erhöht werden.

## Patentansprüche

1. Verfahren zur Aufarbeitung von Mutterlaugen aus der Herstellung von Dihydroxydiarylalkanen durch Umsetzung von Phenolen mit Ketonen und Isoalkenylphenolen an sauren Katalysatoren bei dem
1. aus der ausgeschleusten Mutterlauge Phenol abdestilliert wird, der nach Abdestillieren von Phenol erhaltene Sumpf mit einem basischen Katalysator versetzt und in einer Reaktivrektifikation kontinuierlich gespalten wird in Phenol und Isoalkenylphenol, die als Destillat über Kopf gehen, und in einen Sumpf,
2. der Sumpf mit einem sauren Katalysator vermischt und sauer gestellt wird und in eine zweite Reaktivrektifikation kontinuierlich eingetragen und gespalten wird in Phenol und einen zu entsorgenden Sumpf,
3. Phenol aus Schritt 1 und 2 und Isoalkenylphenol, gegebenenfalls nach einer weiteren Reinigung, in die Reaktion zurückgeführt werden.

## Claims

1. Process for working up mother liquors from the, production of dihydroxydiarylalkanes by reacting phenols with ketones and isoalkenylphenols on acid catalysts in which
1. phenol is removed from the discharged mother liquor by distillation, the bottoms obtained once the phenol has been removed by distillation are combined with a basic catalyst and continuously decomposed in a reactive rectification stage into phenol and isoalkenylphenol, which pass overhead as the distillate, and bottoms,
2. the bottoms are mixed with an acid catalyst and acidified and continuously introduced into a second reactive rectification stage and decomposed into phenol and bottoms to be discarded,
3. phenol from stages 1 and 2 and isoalkenylphenol, optionally after further purification, is returned to the reaction.

## Revendications

1. Procédé de traitement des lessives-mères évacuées lors de la préparation de dihydroxydiarylalcanes par réaction de phénols avec des cétones et des isoalcénylphénols sur des catalyseurs acides, dans lequel
1. des lessives-mères évacuées, on distille le phénol, on ajoute au fond obtenu après distillation du phénol, un catalyseur basique et on le clive en continu dans une rectification réactive en phénol et isoalcénylphénol, qui passent en tête comme distillat, et en un fond,
2. le fond est mélangé à un catalyseur acide et ajusté acide, introduit en continu dans une deuxième rectification réactive et clivé en phénol et en un fond éliminé,
3. le phénol des étapes 1 et 2 et l'isoalcénylphénol, éventuellement après une autre purification, sont réintroduits dans la réaction.
